# EUROPÄISCHE PATENTANMELDUNG

(11) **EP 0 558 919 A1**
(43) Veröffentlichungstag der Anmeldung: **08.09.1993**
(21) Anmeldenummer: 93101267.8
(22) Anmeldetag: 28.01.1993
(51) Int. Cl.: F24F 3/16, B01D 46/10

(54) **Verfahren und Vorrichtung zum Reinigen eines Filters**

(30) Priorität: 02.06.1992 DE 4218090
(71) Anmelder: Adam, Jakob, D-35392 Giessen (DE); AL-KO THERM GmbH Maschinenfabrik, D-89343 Jettingen-Scheppach (DE)
(72) Erfinder: Jakob, Adam, D-6300 Giessen (DE); Kammerer, Franz, D-8909 Münsterhausen (DE)
(74) Vertreter: Munk, Ludwig, Dipl.-Ing.

(57) **Zusammenfassung**

Zum Reinigen eines Filters, insbesondere eines in einen Strömungskanal eingebauten Filters, wie des Luftfilters einer Klimaanlage, wird das Filter in sporadischen Abständen mit einem flüssigen reinigungsmittel beaufschlagt, das zweckmäßig erst nach einer gewissen Einweichzeit abgelassen wird und mit dem die vom Filter gelösten Verunreinigungen abgeführt werden.

## Beschreibung

Die heute zur Anwendung kommenden Luftfilterkonstruktionen für Klimaanlagen sind gesundheitlich sehr bedenklich, da sie Schadstoffe, Schmutz und Keime speichern und zu einer Geruchsbelästigung führen. Der Transport der Außenluft durch die im Luftfilter gespeicherten Verunreinigungen führt zu einer Geruchsbelastung und zu Klagen über unnatürliche Raumluft. Die bisherigen Systemlösungen zur Regeneration bzw. kontinuierlichen Reinigung von Luftfiltern sind unbefriedigend.

### Aufgaben der Erfindung

Die Erfindung stellt sich die Aufgabe, Luftfilter von Klimaanlagen stündlich, täglich oder wöchentlich ohne aufwendige und bewegliche Teile in sehr einfacher Weise automatisch zu reinigen sowie das Luftfilter zu desinfizieren.

### Lösung der Aufgabe

Die Aufgabe der Erfindung wird dadurch gelöst, indem das Luftfiltermaterial abwechselnd stündlich, täglich oder wöchentlich mit einem flüssigen Reinigungsmittel überflutet wird, so daß sich die Schmutzpartikel in einer Reinigungswanne lösen und beim Entfluten abgeführt werden.

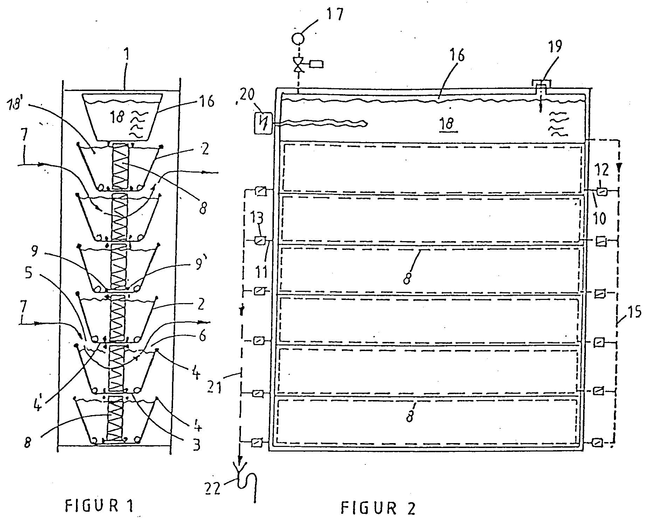

### Aufbau der Erfindung, Figur 1 + 2

In einem Luftkanalstück (1) sind Flüssigkeitsauffangwannen (2) übereinander angeordnet, so daß die Unterkante (3) der Wanne (2) mit der Oberkante der Wanne (4) bündig ist. Hierdurch entsteht eine Lufteintrittsöffnung (5) und eine Luftaustrittsöffnung (6), durch die die Außenluft (7) hindurchströmen kann.
Zwischen den beiden Unterkanten der Wanne (4) + (4') sind Luftfilterkassetten (8) senkrecht angebracht, die über eine aufblasbare Gummileiste (9) + (9') abgedichtet sind. Die Wanne (2) besitzt Flüssigkeitseintrittsstutzen (10) für den Eintritt und Flüssigkeitsaustrittsstutzen (11) für den Austritt des flüssigen Reinigungsmittels. Die Eintrittsstutzen (10) sind mit Magnetventilen (12) und die Austrittsstutzen (11) mit Magnetventilen (13) bestückt, die automatisch nach Programm auf-und zugefahren werden. Die Magnetventile (12) sind über eine Flüssigkeitsrohrleitung (15), z.B. aus Kunststoff, an einen Tank (16) angeschlossen, der über der Wanne (2) angeordnet ist.
In dem Tank (16) wird über die Stadtwasserleitung (17) Wasser (18) eingefüllt, das über dem Stutzen (19) mit Reinigungsmittel beschickt wird. Das mit Reinigungsmittel versetzte Wasserbad (18) kann über eine Heizeinrichtung (20) auf die gewünschte bzw. erforderliche Temperatur erwärmt werden.

Die Magnetventile (13) sind an eine Ablaufleitung (21) angeschlossen, die in den Bereich der Entwässerung (22) geführt wird.

### Betriebsweise, Figur 1 + 2

Strömt Außenluft (7) durch die Luftfilterkassetten (8), dann befindet sich vorzugsweise kein flüssiges Reinigungsmittel (18') in den Wannen (2), höchstens jedoch nur in einer der Wannen (2). Bei Betriebsende täglich oder wöchentlich, wenn die Außenluftzufuhr (7) unterbrochen ist, werden bei geschlossenen Magnetventiler (13) und geöffneten Magnetventilen (12) die Wannen (2) mit flüssigen Reinigungsmitteln (18) aus dem Tank (16) soweit gefüllt, daß das Reinigungsmittel (18') bis zur Oberkante (4) ragt und die Luftfilterkassetten (2) mit Reinigungsmittel (18') überflutet sind. Das Reinigungsmittel (18) bzw. (18') kann über die Heizeinrichtung (20) auf die erforderliche Temperatur erwärmt werden. Noch günstiger ist es, wenn das Reinigungsmittel (18') in der Wanne (2) über eine Heizeinrichtung vor Ort erwärmt wird. Die Luftfilterkassetten (8) können sich eine oder mehrere Stunden im Reinigungsmittel (18') befinden, so daß eine ausreichende Reinigung und Desinfektion zustande kommt. Ist der Reinigungsvorgang abgeschlossen und strömt Außenluft (7) über die Luftfilterkassetten (8), dann werden vorher die Magnetventile (13) automatisch nach Programm geöffnet, so daß das Reinigungsmittel (18') und die enthaltenen Verunreinigungen über die Rohrleitung (21) und die Entwässerung (22) abfließen kann. Die übereinander angeordneten Wannen (2) können auch jede einzeln für sich mit Reinigungsmittel (18') beschickt und entleert werden.
Diese Betriebsweise ist dann erforderlich, wenn die LuftfilterKassetten (3) während des Betriebes der Klimaanlage in Teilabschnitten gereinigt werden sollen. In diesem Fall muß jedoch das Reinigungsmittel (18') gesundheitlich unbedenklich sein.

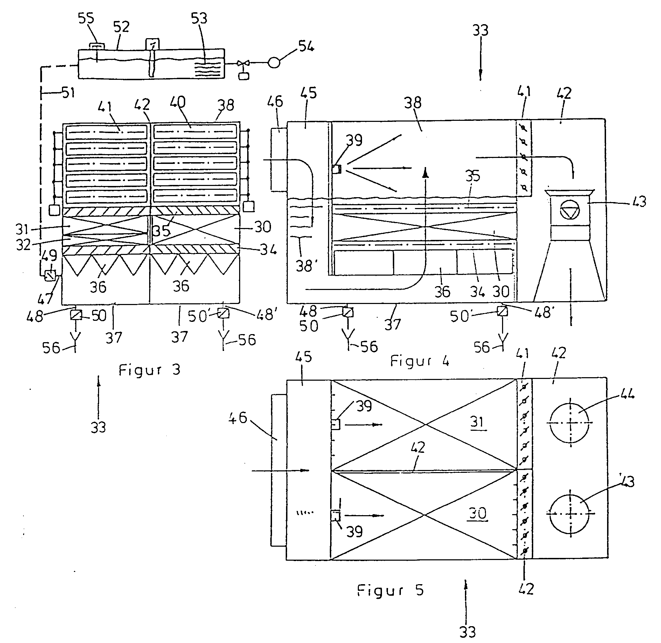

### Aufbau, Figur 3, 4, 5

Sollen auch die Luftaufbereitungsstufen, Kühler, Lufterhitzer, WRG-Batterie und Tropfenabscheider gereinigt werden, weil an diesen vor allem durch Taupunktunterschreibung bzw. insbesondere Schwitzwasseranfall und den Feinstaub, der durch das Luftfilter hindurchgeht, Ablagerungen entstehen, die einen unangenehmen Geruch (z.B. Kühlergeruch) verursachen, dann müssen auch die Luftaufbereitungskomponenten kontinuierlich gereinigt werden. Hierzu ist es erforderlich, die Luftfilter und die Gerätekomponenten anders anzuordnen als bisher. Um einen Beipaßbetrieb zwischen den Luftaufbereitungsstufen, Kühlung einerseits und Vorwärmung mit Wärmerückgewinnung andererseits, zu ermöglichen, ist der Luftkühler (30) neben dem Lufterhitzer (31) und der WRG-Batterie (32) nebeneinander und waagerecht in dem Gehäuse des Klimazentralgerätes (33) angeordnet. Unterhalb des Luftkühlers (30) sowie der WRG-Batterie (32) ist ein unterer Tropfenabscheider (34) und oberhalb des Luftkühlers (30) und des Erhitzers (31) ein oberer Tropfenabscheider (35) angeordnet. Vor dem unteren Tropfenabscheider (34) befindet sich ein Luftfilter (36), das in eine wasserdichte Wanne (37) hineinragt. Über dem oberen Tropfenabscheider (35) ist eine Luftführungskammer (38) angeordnet, die mit Dampfbefeuchtungsdüsen (39) bestückt ist. An die Luftführungskammer (38) ist eine rechte Luftregelklappe (40) und eine linke Luftregelklappe (41) angeordnet, wobei sich zwischen den beiden Luftregelklappen (40) + (41) eine Trennwand (42) befindet, die den Luftkühler (30) von dem Lufterhitzer (31) und der WRG-Batterie (32) luftseitig trennt.

Neben den Luftregelklappen (40) + (41) befindet sich eine Ventilatorkammer (42), die mit Axialventilatoren (43) + (44) oder Radialventilatoren ausgerüstet ist. Links neben der Luftführungskammer (38) ist eine Außenluftkammer (45) angebracht, die einen Lufteintrittsstutzen (46) besitzt. An der wasserdichten Wanne (37) sind Anschlußstutzen (47) + (48) + (48'), an denen sich Magnetventile (49) + (50) + (50') befinden.
Das Magnetventil (49) ist über eine Rohrleitung (51) mit einem Wassertank (52) verbunden, der mit einem flüssigen Reinigungsmittel (53) versehen ist. Der Wassertank (52) ist an eine Frischwasserleitung (54) angeschlossen und mit einem Einfüllstutzen (55) für Reinigungsmittel versehen.

### Betriebsweise

Bei Stillstand der Axialventilatoren (43) sind die Magnetventile (50) + (50') geschlossen und das Magnetventil (49) geöffnet. Das Reinigungsmittel (53) des Wassertanks (52) fließt in die Wanne (37) ein und überflutet das Luftfilter (36), die Tropfenabscheider (34) + (35), den Lufterhitzer (31) und die WRG-Batterie (32) bis zur Unterkante der Luftregelklappen (40) + (41) und den Luftansaugstutzen (36) und den Kühler (30). Das Reinigungsmedium (53), das auch Desinfektionsmittel enthalten kann, löst während des Stillstandes der Klimaanlage ca. 8 - 12 Stunden/Tag die Schmutzpartikel in den Komponenten (34) + (30) + (31) + (32) + (36) + (35) des Klimazentralgeräts (33) und tötet Keime ab. Vor Betriebsbeginn werden die Magnetventile (50) + (50') geöffnet und das Magnetventil (49) geschlossen. Das mit Schmutzpartikeln versehene Reinigungsmittel (38') fließt über die Magnetventile (50) + (50') ab und wird über die Entwässerung (56) abgeführt. Der Reinigungsprozeß kann täglich, wöchentlich oder monatlich vorgenommen werden. Der energiesparende Betrieb des Klimagerätes wird dadurch sichergestellt, daß die Luftregelklappen (40) + (41) in Abhängigkeit vom Heiz- und Kühlbedarf nur soviel Außenluft über den Luftkühler (30) leiten, wie zur Abkühlung der Außenluft erforderlich ist. Im Heizbetrieb läuft der Vorgang ähnlich ab. Über dem Lufterhitzer (31) und die WRG-Batterie (32) strömt nur soviel Außenluft, wie zur Erwärmung der Außenluft erforderlich ist. Die Regelung der Luftmengenanteile wird über die Luftregelklappen (40) + (41) vorgenommen. Im Kühlfall ist das Kühlerventil offen und das Lufterhitzerventil zu. Im Heizfall hingegen ist das Kühlerventil zu und das Heizventil auf und wird nur um den Betrag geöffnet, wie zur Erwärmung der Außenluft erforderlich ist. Die Folgeregelung zwischen den Luftregelklappen (40) + (41) hat jedoch Vorrang.

Die Veränderung der Druckverluste bei der luftseitigen Regelung über die Luftregelklappen (40) + (41) wird über eine Drehzahlregelung der Axialventilatoren (43) + (44) ausgeglichen.

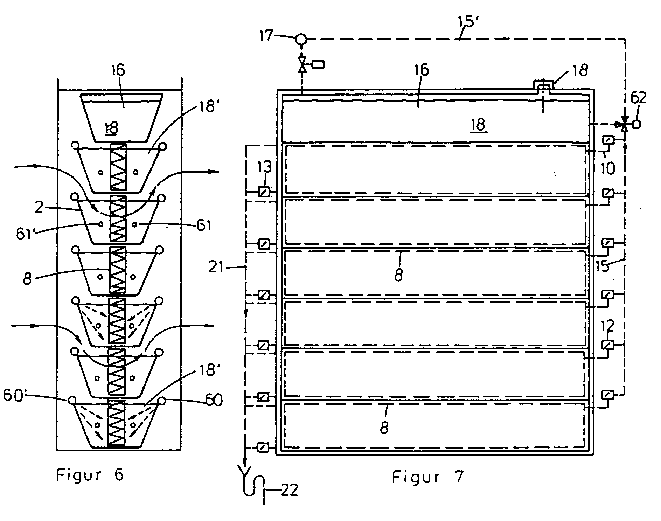

### Aufbau und Betriebsweise, Figur 6 + 7

Wird der Reinigungsturnus zur Reduzierung des Wasserverbrauchs verlängert (z.B. monatliche Reinigung), dann ist die Schmutzspeicherung größer und fester. In diesem Falle ist nach dem Einweichvorgang, d.h. nach dem längeren Verbleiben der Luftfilter (18) im Reinigungsmittel eine intensive Nachspülung mit reinem Spritzwasser von großem Vorteil. Für diesen Fall sind an der linken und rechten Oberkante der Wanne (2) Wasserverteilrohre (60) + (60') angebracht, die mit Spritzwasserdüsen ausgerüstet sind. Die beiden Verteilrohre (60) + (60') sind an das Magnetventil (12) über ein Kreuzstück angeschlossen. Links und rechts von den Luftfilterkassetten (8) verlaufen Heizdrähte (61) + (61') oder Dampfrohre. Die Rohrleitung (15) kann auch an einen Dampferzeuger angeschlossen sein. Nach Stillstand der Klimaanlage wird das Reinigungsmittel (18) aus dem Tank (16) über das Dreiwegeventil (62) und die Magnetventile (12) in die Verteilrohre (60) + (60') eingeleitet. Die Magnetventile (13) sind geschlossen. Die Luftfilter (8) werden vom Reinigungsmittel (18) überflutet. Nach der Einweichzeit von bis zu 10 Stunden lösen sich die Schmutzpartikel. Nach dem Öffnen der Magnetventile (13) und vor der Inbetriebnahme der Klimaanlage wird das Dreiwegeventil (62) umgeschaltet. Über die Rohrleitung (15) und die Verteilleitungen (60) + (60') wird Frischwasser mit verhältnismäßig hohem Druck über Spritzdüsen in Richtung der Luftfilter (8) gespritzt. Damit lösen sich die restlichen Schmutzpartikel in dem Luftfiltermaterial (8) und werden über die Rohrleitung (21) zusammen mit den Frischwasser abgeführt. Das Reinigungsmittel (18) kann über die Heizdrähte (61) + (61') in der gewünschten und zulässigen Weise erwärmt werden. Bei Keramikfiltermaterial kann das Reinigungsmittel (18) in der Wanne (2) bis auf ca. 100 °C erwärmt werden. Nach Entleerung der Wanne (2) können die Heizdrähte (61) + (61') die Luftfilter (8) durch Erwärmung der Umgebungsluft trocknen.

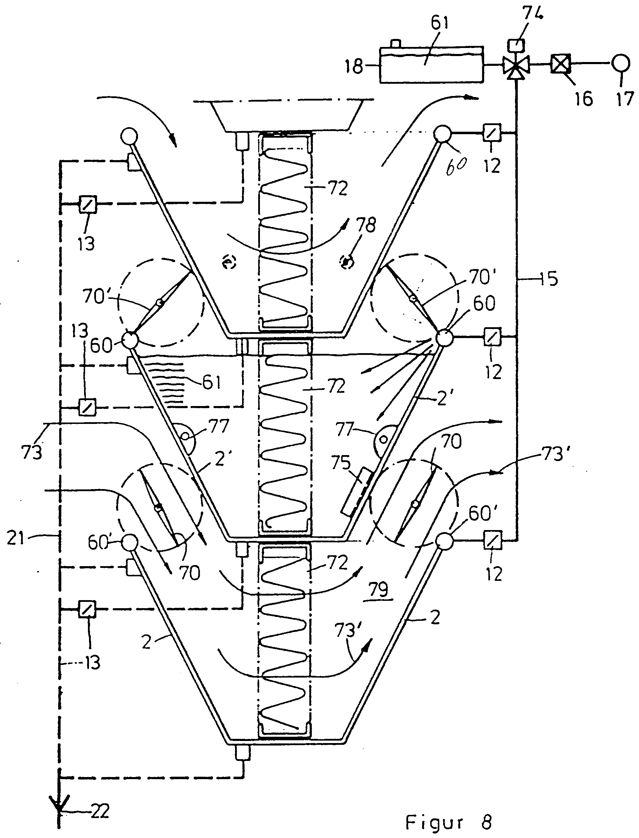

### Aufbau und Betriebsweise, Figur 8

Von großer Bedeutung für die Reinigung von Luftfiltermedien ist, daß die feinen und engen Fasern durch Druckeinwirkungen (Spritzwasser oder Druckluft) nicht beschädigt werden. Hinzu kommt, daß möglichst keine aggressiven Reinigungsmittel verwendet werden sollten. Hierzu ist es erforderlich, den Einweichvorgang zu verlängern und in vielen Fällen sogar während des Betriebes der Klima anlage vorzunehmen. Dabei muß jedoch gewährleistet sein, daß sich die Schmutzpartikel lösen. Die Ausführung hierzu ist in Figur 8 dargestellt.
Zwischen den einzelnen, übereinander angeordneten Reinigungswannen (2) + (2') sind Absperrklappen (70) + (70') auf der rechten und linken Seite der Wannen (2) + (2') drehbar angeordnet und mit Stellmotoren versehen. Ist die Wanne (2') mit einem vor allem milden Reinigungsmittel (61) gefüllt, dann sind die Klappen (70') rechts und links von der Wanne (2') geschlossen. Der Luftdurchgang durch das Luftfilter (72) ist dann unterbrochen. Die Wanne (2) und die anderen Wannen sind dann nicht mit Reinigungsmittel (61) gefüllt. Die Luftabsperrklappen (70) an den beiden Seiten der Wanne (2) sind offen. Damit kann die ungereinigte Außenluft (73) links in die Wanne (2) einströmen, vom Luftfilter (72) gereinigt werden und rechts als gereinigte Außenluft (73') ausströmen.
Das Reinigungsmittel (61) gelangt bei geschlossenen Absperrklappen (70') vom Tank (16) über das Dreiwegeventil (74), die Rohrleitung (15), das geöffnete Magnetventil (12) in das Sprührohr (60) und füllt die Wanne (2') mit Reinigungsmittel (61) bis zur Oberkante des Luftfilters (72) auf. Die Luftfilterkassette (72) verbleibt dann mehrere Stunden im milden Reinigungsmittel (61).

Zur intensiveren Reinigung kann außerdem das Reinigungsmittel (61) in der Wanne (2') über ein Ultraschallgerät (75) in Schwingungen versetzt werden, so daß sich auch die letzten Schmutzpartikel lösen, ohne daß das feine Luftfiltermaterial (61) beschädigt oder so verändert wird, daß sich der Abscheidegrad verschlechtert. Nach dem mehrstündigen Einweichvorgang wird das Magnetventil (13) geöffnet. Das Reinigungsmittel (61) fließt mit den enthaltenen Verunreinigungen über die Rohrleitung (21) zur Entwässerung (22).
Nach der Entleerung der Wanne (2') strömt Frischwasser über das Frischwassernetz (17), das Dreiwegeventil (74), das Magnetventil (12) zum Sprührohr (60) und wird über dieses in Richtung des Luftfilters (72) geleitet. Das Frischwasser gelangt nach dem Spülvorgang über das Magnetventil (13), die Leitung (21) zur Entwässerung (22). Das Frischwasser kann außerdem je nach Luftfiltermaterial )72) von dem Wassererwärmer (76) auf ca. 60 °C oder 95 °C erwärmt werden. Die Erwärmung kann auch vor Ort über Heizdrähte (78) vorgenommen werden. Nach dem Spülvorgang oder sogar während dem Einweichvorgang können UVC-Lampen (Entkeimungslampen) das Luftfilter (72) bestrahlen und Keime abtöten.
Da der Luftraum (79) über den Klappen (70') geschlossen ist, kann das Luftfilter (72) auch über die UVC-Lampen (77) entkeimt und erwärmt bzw. getrocknet werden. Die eingangs beschriebene Betriebsweise:

| | |
|---|---|
| Einweichvorgang | 2 bis 10 Stunden |
| Spülvorgang | 5 bis 15 Minuten |
| Trockenvorgang | 5 bis 15 Minuten (bei Entkeimung mit Lampen ca. 1 Stunde) |

stellt sicher, daß das Luftfiltermaterial ohne Beschädigung täglich oder wöchentlich einwandfrei gereinigt und desinfiziert wird, ohne daß die Klimaanlage oder lufttechnische Anlage außer Betrieb genommen werden muß.

Beim vorstehend beschriebenen Beispiel wird das Reinigungsmittel der Wanne 2 durch ein jeweils zugeordnetes, mit auf das Luftfilter 72 ausgerichteten Sprühdüsen versehenes Sprührohr 60 zugeführt. Bei Beginn des Reinigungsvorgangs wird das Luftfilter 72 daher mit Reinigungsmittel besprüht. Sofern dieses in der Wanne 2 aufgefangen und gesammelt wird, wird das Luftfilter 72 langsam überflutet. Im Einweichvorgang ist hier daher ein Lockerungsvorgang durch Besprühen vorgeordnet. In einfachen Fällen kann ein Einweichvorgang entfallen. Dabei genügt eine Reinigung durch über eine vorgegebene Zeit sich erstreckendes Besprühen. Zur Intensivierung der Reinigung kann dabei der Sprühvorgang pulsierend gestaltet werden. Ebenso wäre es denkbar, die Düsen hin- und herzubewegen. Die Wanne 2 fungiert beim Sprühen als Spritzschutz und im geöffneten Zustand als Ableiteinrichtung. Ein Spülvorgang kann sich in jedem Falle anschließen.

Eine Vor- bzw. Hauptreinigung durch Besprühen kann selbstverständlich auch im Zusammenhang mit den anderen Ausführungsbeispielen Anwendung finden.

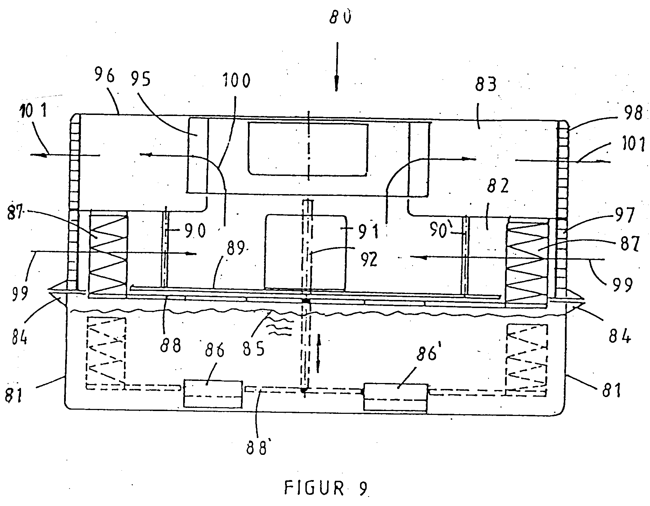

### Aufbau und Betriebsweise Figur 9

Die Grundlage der Erfindung kann auch bei den konventionellen Luftreinigungsgeräten für Büros und Wohnungen zum Einsatz kommen, da gerade bei diesen Geräten durch Schmutzspeicherung im Luftfiltermaterial erhebliche Geruchsbelästigungen entstehen, die dazu führen, daß sie außer Betrieb genommen werden. Der Druckverlust steigt mit zunehmender Schmutzspeicherung erheblich an, die Zuluftmenge vermindert sich extrem. Der Austausch des verdreckten Luftfilters ist oft nicht einfach und wird außerdem von Hausfrauen nicht gerne vorgenommen. Pilzbefall kann außerdem gesundheitlich nachteilige Folgen haben. Der Luftreiniger (80) in Kompaktbauweise besteht vorzugsweise aus einem runden Reinigungsbehälter (81), der nach oben hin offen ist und einen Auflagerahmen (84) besitzt, auf dem das Filterteil (82) und darüber das Ventilatorteil (83) aufgesetzt ist. Das Filterteil (82) und das Ventilatorteil (83) lassen sich gemeinsam von dem Reinigungsbehälter (81) abheben. Dies ermöglicht eine leichte Füllung mit Reinigungsmittel (85). In dem Reinigungsmittel (85) kann auch ein Bioabsorber beigemischt sein, wie dies z.B. beim Venta-Gerät üblich ist. Auf dem Boden des Reinigungsbehälters (81) sind Ultraschallschwinger (86) angebracht, die das Reinigungsmittel (85) in Schwingungen versetzen. Das Filterteil (82) ist mit einem Filterring (87) ausgerüstet, der von einer Scheibe (88) getragen wird. Das Reinigungsteil (81) wird von dem Filterteil (82) über eine runde Scheibe (89) getrennt. Sie ist über Bolzen (90) an der oberen Wand des Filterteils (82) befestigt. In dem Zentrum der Scheibe (89) befindet sich ein Stellmotor (91), z.B. Hubmagnet, dessen Verstellstange (92) mit der Scheibe (89) verbunden ist. Der Boden (93) des Filterteils (82) ist mit einer Öffnung (94) versehen, über der ein Radialventilator (95) angeordnet ist. Der Radialventilator (95) ist an dem oberen Abschlußdeckel (96) befestigt. Das Filterteil (82) und das Ventilatorteil (83) sind umlaufend mit kreisrunden Leitlamellen (97) + (98) versehen.

### Betriebsweise:

### Luftreinigung

Der Radialventilator (95) saugt über das runde Gitterband (97) verschmutzte Raumluft (99) an und fördert diese über den Luftfilterring (87). Die gereinigte Raumluft (100) strömt als Zuluft (101) über das Gitterband (98) vorzugsweise radialsymmetrisch in den Raum.

### Reinigung des Luftfiltermaterials

Der Radialventilator (95) wird vor allem nachts für einige Zeit stillgesetzt. Der Stellmotor (91) drückt die Scheibe (88) und damit auch den Luftfilterring (87') in das Reinigungsbad (85). Der anschließende Einweichvorgang reinigt den Luftfilterring (87) bzw. (87'). Der Reinigungsprozeß wird gesteigert, wenn die Ultraschallschwinger (86) + (86') in Betrieb genommen werden.

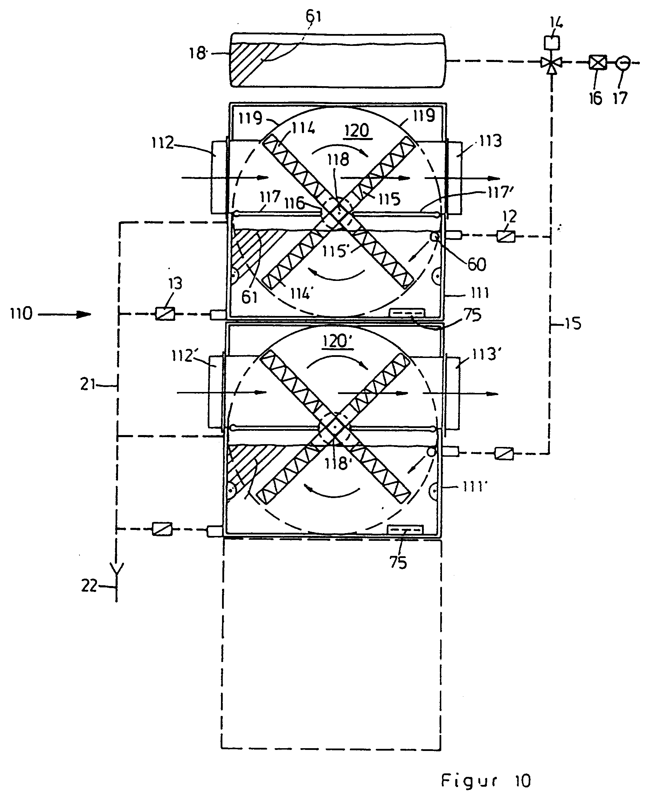

### Aufbau und Arbeitsweise Figur 10

Für viele Anwendungsfälle, insbesondere für Lüftungsanlagen, die un unterbrochen in Betrieb gehalten werden, ist eine kontinuierliche Reinigung unerläßlich. Sie ist zwar nach der Ausführung gemäß Figur 8 mit den Luftabsperrklappen (70) + (70') möglich, jedoch etwas komplizierter. Das Regenerationsluftfilter kann einfacher aufgebaut sein, die Abmessungen sind jedoch größer.
Eine weitere Verbesserung ergibt sich, wenn man anstelle der einstufigen Luftfilterung eine zweistufige vorsieht. Die zweistufige Regenerationsluftfilteranlage (110) besteht aus einze übereinander angeordneten Blechkanälen (111) + (111'), die auf der linken Seite Lufteintrittsöffnungen (112) und auf der rechten Seite Lufteintrittsöffnungen (113) besitzen, die etwas kleiner sind als die halbe Höhe des Blechkanals (111) aus Edelstahl. Die Luftöffnungen (112) + (113) erstrecken sich über die ganze Länge des Blechkanals (111).
Die Endstücke der Blechkanäle (111) + (111') sind wasserdicht verschlossen, aber als Enddeckel lösbar. Im Zentrum des Luftkanals (111) + (111') ist ein Vierkantstück (118) angeordnet, das in den Enddeckeln drehbar gelagert ist. Auf dem Vierkantrohr (118) sind unter einem Winkel von 45 Grad Luftfilterkassetten (114) + (114') sowie (115) + (115') befestigt. Die Filterkassetten (114) + (114') dienen als Vorfilter mit geringerem Abscheidegrad, während die Luftfilterkassetten (115) + (115') als Nachfilter mit höherem Abscheidegrad dienen. Das Vierkantrohr (118) ist mit einem Drehstellmotor (116) versehen, der die Filterkassetten (114) + (114') sowie (115) + (115') um 180 Grad verdrehen kann. Zwischen den oberen Filterkassetten (114) + (115) und den unteren Filterkassetten (114') + (115') sind Trennbleche (117) + (117') angeordnet, die an dem Vierkantrohr (118) befestigt sind und bündig zu den Unterkanten der Öffnungen (112) + (113) verlaufen.
Oberhalb der Luftfilterkassetten (114) + (115) verläuft ein Leitblech (119), das die Luftfilterkassetten (114) + (115) luftdicht abdichtet. Das Unterteil der Blechkanäle (111) + (111') ist mit Reinigungsmittel (61) oder Frischwasser gefüllt, das die Luftfilterkassetten (114') + (115') überflutet. In den Blechkanälen (111) + (111') im Bereich des Reinigungsmittels (61) sind Ultraschallgeräte (75) und UVC-Entkeimungslampen (77) angeordnet. Die Fülleitung (15) und Entleerungsleitung (21) sind mit den Armaturen und Zusatzeinrichtungen genauso ausgerüstet wie in der Figur 8 dargestellt und beschrieben.

### Betriebsweise Figur 10

Die ungereinigte Außenluft (73) strömt über die Öffnung (112) ein und durchströmt anschließend das Vorfilter (114) und durch das Nachfilter (115), das vorher im Reinigungsmittel (61) im Unterteil des Blechkanals (111) + (111') gereinigt wurde.
Die gereinigte Außenluft (73') wird nach dem Reinigungsprozeß von dem Klimazentralgerät und den Luftkanälen in die Büroräume geleitet. Nach einer gewissen Zeit, die von der Außenluftverschmutzung und der erwünschten Luftqualität bestimmt wird, verdreht der Drehstellmotor (116) die Luftfilterkassetten (114) + (115) in das Reinigungsbad (61) im Unterteil des Blechkanals (111). Gleichzeitig werden die vom Reinigungsbad (61) gereinigten Filterkassetten (114') + (115') nach oben in den Luftführungsraum (120) verdreht. Dieser Vorgang wiederholt sich in kürzeren oder längeren Zeitabständen während der ganzen Betriebszeit der Lüftungs- oder Klimaanlage. Die Reinigungs-, Spül-, Desinfektions- und Trocknungsvorgänge wurden im Detail in Figur 8 beschrieben. Anstelle einer Filterkassette (72) werden jedoch zwei Filterkassetten (114') + (115') gereinigt.

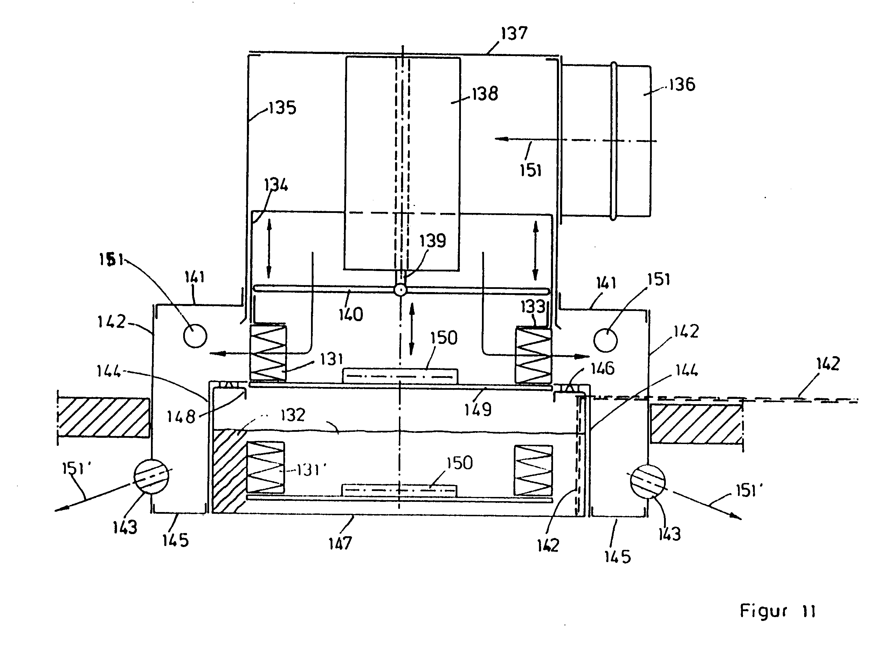

### Aufbau und Arbeitswiese Figur 11

Eines der Hauptprobleme der Klimatisierung ist es, daß die Zuluftkanalsysteme verstaubt und verschmutzt sind und somit eine ideale Brutstätte von Keimen, Bakterien, Pilzen und Allergenen bilden. Das gilt vor allem für bestehende Klima- und Lüftungsanlagen, die schon sehr lange in Betrieb sind. In solchen Fällen nutzt eine sehr gute Vorfilterung nicht viel; die Zuluft muß dann endständig, d.h. am Zuluftauslaß vor dem Eintritt in den Raum, gereinigt werden.
Luftfiltersysteme, die sich nicht in kurzen Zeitabständen regenerieren lassen, sind jedoch sehr problematisch, weil sie Schmutz und Geruchspartikel speichern und somit die Zuluft und Raumluft mit Gerüchen, Allergenen und Keimen belasten. In der Figur 11 ist ein Deckenzuluftauslaß (130) dargestellt, der einen endständigen Luftfilterring (131) besitzt, der in einem Reinigungsbad (132) kontinuierlich gereinigt werden kann.
Der Luftfilterring (132) ist über eine Blechscheibe (133) an einem in der Höhe verschiebbaren Rohrstück (134) befestigt. Das Rohrstück (134) befindet sich in einem Verteilrohr (135), das einen Zuluftstutzen (136) und einen Abschlußdeckel (137) besitzt. An den Abschlußdeckel (137) ist ein Stellmotor (138) montiert, dessen Verstellstange (139) an einem Rohrkreuz (140) befestigt ist, das mit dem Rohrstück (134) verbunden ist. Das Verteilrohr (135) wird von einer Scheibe (141) umschlossen. An der Scheibe (141) ist ein rundes Blechrohr (142) befestigt, das umlaufend mit beweglichen Kugeldüsen (143) oder Zylinderdüsen bestückt ist.
Im Blechrohr (142) ist ein Innenrohr (144) plaziert, das über eine Lochscheibe (145) an dem Blechrohr (142) befestigt ist. Das Innenrohr (1444) ist oben umgebordelt. An dem Bördelring sind Haftmagnete (146) angebracht. In dem Innenrohr (144) ist ein Reinigungskopf (147) angeordnet, dessen oberer Bördelring (148) an den Haftmagneten (146) anliegt und von diesen gehalten wird.
Der Luftfilterring (131) ist mit einer Blechscheibe (149) fest verbunden. Im Zentrum der Blechscheibe (149) ist eine Ultraschallscheibe (150) installiert.

### Betriebsweise Figur 11

Die Zuluft (151) strömt über den Luftfilterring (131) und wird von diesem gereinigt und strömt als gereinigte Zuluft (151') radialsymmetrisch über die Kugeldüsen in den Raum. Bei Stillstand der Klimaanlage oder unterbrochener Luftzufuhr (z.B. Verschluß des Zuluftstutzens (136) mit einer Absperrklappe) verschiebt der Stellmotor (138) über die Verstellstange (139) das Rohrkreuz (140), das Rohrstück (134) nach unten. Damit gelangt der an dem Rohrstück (134) befestigte Luftfilterring (131) zusammen mit der Scheibe (149) und der Ultraschallscheibe (150) in das Reinigungsbad (132) des Reinigungstopfes (147). Das Reinigungsbad (132), das mit Bioabsorber versetzt sein kann, wird über die Ultraschallscheibe (150') zusammen mit der Scheibe (149) und dem Luftfilterring (131) in Schwingungen versetzt, so daß sich infolge des Einweichvorganges und der Schwingungen die Schmutzpartikel von dem Luftfilterring (131) lösen. Nach dem Einweich- und Reinigungsvorgang (durch Ultraschall), der 10 Minuten bis 10 Stunden dauern kann, wird der Luftfilterring (131) langsam aus dem Reinigungsbad (132) gehoben.
Der Luftfilterring (131) wird außerdem eine Zeitlang direkt über dem Reinigungsbad (132) gehalten, bis die Reinigungsflüssigkeit (132) abtropft. Danach hebt der Stellmotor (138) den Luftfilterring (131) in die Lüftungsbetriebsstellung. Diese ergibt sich, wenn die Scheibe (149) mit den Haftmagneten (146) in einer Flucht ist. Danach werden die UVC-Entkeimungslampen (151) eingeschaltet, die den Luftraum vor dem Luftfilterring (131) erwärmen und diesen entkeimen und trocknen.
Der Austausch des Reinigungsbades (132) erfolgt im wöchentlichen oder monatlichen Rhythmus, indem mittels einer Hubeinrichtung der Reinigungstopf (147) von dem Innenrohr (144) herausgenommen und mit einem neuen Reinigungstopf (147) versehen wird, der mit einem neuen Reinigungsbad (132) versehen ist. Das Reinigungsbad (132) kann aber auch über eine flexible Leitung (152), die bis in den Flur oder in die Naßräume reicht, entleert und neu gefüllt werden.

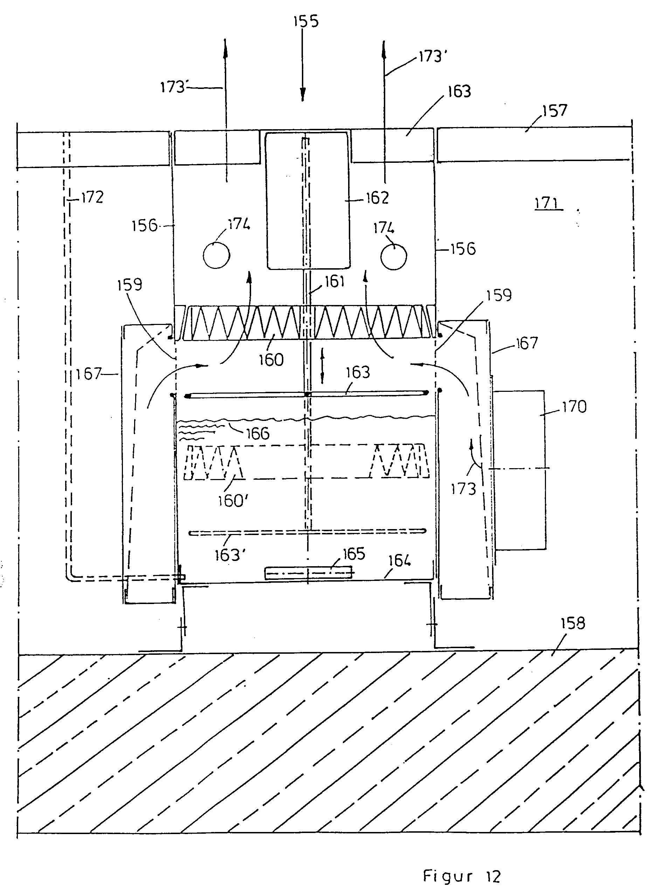

### Aufbau und Betriebsweise Figur 12

Bodenluftsysteme sind lufthygienisch besonders bedenklich, da der Bürostaub (bei Fensterlüftung auch Straßenstaub) bei Lüftungsstillstand durch Querströmungen (Druckdifferenz z.B. Luv + Lee) in die Bodenauslässe gelangt. Die Beschwerden über die Luftqualität sind bei diesem Luftführungssystem besonders groß. Der Bodenluftauslaß (155) besteht aus einem inneren Luftausblasrohr (156), das von der Bodenplatte (157) bis kurz vor die Betondecke (158) reicht. Das Luftausblasrohr (156) ist etwa in der Mitte mit umlaufenden Bohrungen (159) versehen. Über den Bohrungen (159) ist eine Luftfilterscheibe (160) angeordnet, die über eine Verstellstange (161) mit einem Stellmotor (162) verbunden ist. Der Stellmotor (162) ist an dem Drallauslaß (163) befestigt, der von dem Luftausblasrohr (156) aufgenommen wird. An der nach unten verlängerten Verstellstange (161) ist eine Scheibe (163) angebracht, die bündig zu den unteren Bohrungen (159) verläuft und ca. 3 - 10 mm im Durchmesser kleiner ist als das Luftausblasrohr (156). An dem unteren Ende des Ausblasrohres (156) ist ein wasserdichter Abschlußdeckel (164) angebracht, auf dem eine Ultraschallscheibe (165) aufgelegt ist. Das im unteren Bereich wasserdichte Ausblasrohr (156) ist mit einem Reinigungsbad 8166) ausgefüllt, das bis ca. 10 - 15 mm unterhalb der Bohrungen (159) reicht.
Das Luftausblasrohr (156) ist von einem umlaufenden Luftverteilrohr (167) umgeben, das von einer oberen Scheibe (168) und einer unteren Scheibe (169) luftdicht verschlossen ist. Das Luftverteilrohr (167) besitzt einen Stutzen (170) zum Anschluß an ein Zuluftrohr im Doppelbodenhohlraum (171). Das Luftausblasrohr (156) kann mit einer flexiblen Füll- und Entleerungsleitung (172) versehen sein, die bis in die Bodenplatte (157) reicht.

### Betriebsweise Figur 12

Beim Lüftungsbetrieb befindet sich die Luftfilterscheibe (160) oben über den Bohrungen (159). Die Zuluft (173) strömt über das Luftverteilrohr (167) sowie die Bohrungen (159) und durch die Luftfilterscheibe (160) und wird von dieser gereinigt. Die gereinigte Zuluft (173') gelangt über den Drallauslaß (163) in den Raum.
Bei unterbrochener Luftzufuhr (173) drückt der Stellmotor (162) über die Verstellstange (161) die Luftfilterscheibe 8160) in das Reinigungsbad (166). Der Luftfilterring (160') wird im Reinigungsbad (166) über einen längeren Einweichvorgang gereinigt. Der Reinigungsgrad wird über die Ultraschallscheibe (165) wesentlich gesteigert, indem diese das Reinigungsbad (166) in Schwingungen versetzt. Nach dem Reinigungsbad wird die Luftfilterscheibe (160) vom Stellmotor (162) langsam nach oben gehoben und von den UVC-Entkeimungslampen (174) erwärmt und entkeimt.

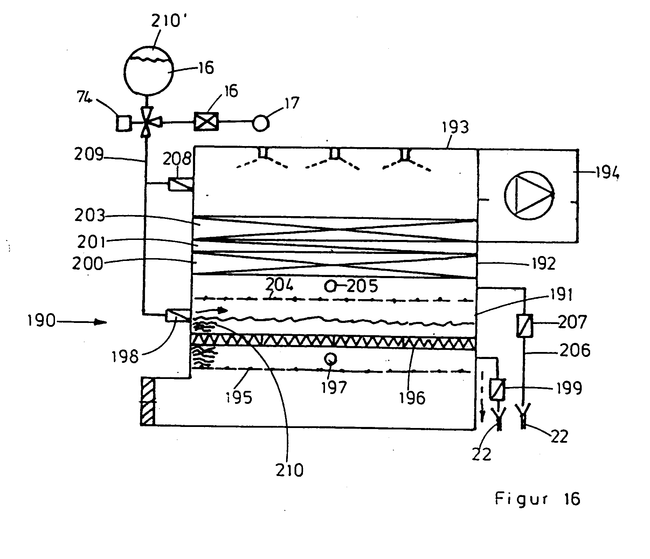

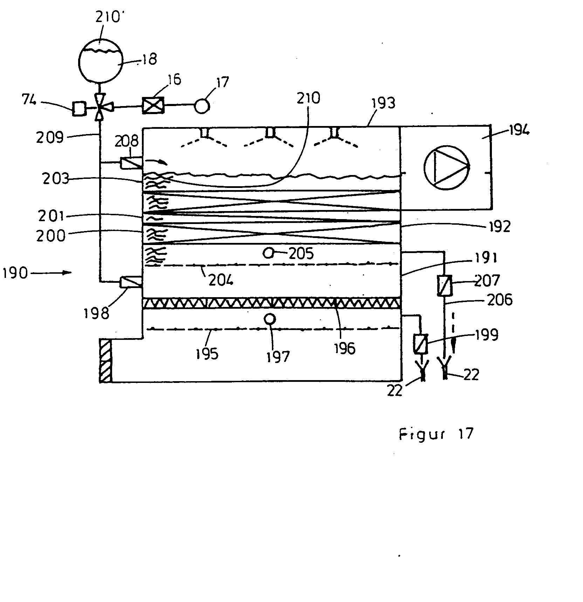

### Aufbau und Arbeitsweise gemäß Figur 16 + 17

Bei der Reinigung der Komponenten eines Klimazentralgerätes nach Figur 3 + 4 + 5 wird eine relativ große Reinigungsmenge zur Überflutung der Komponenten benötigt. Außerdem wird der Außenluftansaug überflutet. Diesen Nachteil kann man ausschalten, wenn nur die einzelnen Komponenten in einem Reinigungsbad gereinigt werden.
Hierzu ist die Ausführung gem. Figur 16 + 17 besonders geeignet.
Das Klimazentralgerät (190) besteht aus einer Luftansaugkammer (191), auf der eine Wärmeaustauscherkammer (192) aufgelegt ist. Über der Wärmeaustauscherkammer (192) ist die Verteilkammer (193) angeordnet, an der eine Ventilatorkammer (194) angeflanscht ist. In der Mitte der Ansaugkammer (191) sind unter wasserdichte Luftregelklappen (195) installiert, die über einen Stellmotor geöffnet oder geschlossen werden können; über den unteren wasserdichten Luftregelklappen (195) sind Luftfilterkassetten (196) angeordnet, die seitlich herausziehbar sind. Zwischen dem Luftfilter (196) und den Luftabsperrklappen (195) sind Ultraschalleinrichtungen (197) plaziert. Oberhalb des Luftfilters (196) ist ein Eintrittmagnetventil (198) und unterhalb ein Austrittmagnetventil (199) angebracht. In der Wärmeaustauscherkammer (192) sind die WRG-Batterie (200), der Vorwärmer (168) und der Luftkühler (203) installiert. Unterhalb der WRG-Batterie (200) ist eine obere wasserdichte Luftabsperrklappe (204) installiert, zwischen der Luftabsperrklappe (204) und der WRG-Batterie (200) befinden sich Ultraschalleinrichtungen (205). Zwischen der WRG-Batterie (200) und den Luftabsperrklappen (204) ist eine Entwässerungsleitung (206) angebracht, in der sich ein Magnetventil (207) befindet. Oberhalb des Luftkühlers (203) ist ein Magnetventil (208) montiert, das an eine Bewässerungsleitung (209) angeschlossen ist. Die Bewässerungsleitung (209) ist über das Dreiwegeventil (74) mit dem Reinigungstank (18) und dem Frischwasseranschluß (17) verbunden.
Beim Reinigungsvorgang sind die Luftabsperrklappen (195) + (204) geschlossen. Soll das Luftfilter (196) gereinigt werden, dann strömt über das Magnetventil (198) bei geschlossenem Magnetventil (199) Reinigungsmittel (210) in die Ansaugkammer (191) über die Absperrklappe (145) ein, bis das Luftfilter (196) überflutet ist. Nach einem längeren Einweichvorgang und der Unterstützung durch die Ultraschalleinrichtung (197), die das Reinigungsmittel (210) in Schwingungen versetzt, wird das Magnetventil (199) geöffnet.
Das Reinigungsmittel (210) entweicht zusammen mit den gelösten Schmutzpartikeln über die Entwässerung (22) in die Kanalisation. Die gleiche Betriebsweise ergibt sich auch bei der Reinigung der Wärmeaustauschkammer (192). Das Reinigungsmittel (210) strömt über das geöffnete Magnetventil (208) bei geschlossenen Absperrklappen (204) in die Wärmeaustauscherkammer (192) und überflutet die Wärmeaustauscher (201) + (202) + (203).
Nach dem Einweichvorgang und dem Reinigungsvorgang mit der Ultraschalleinrichtung (205) wird bei geschlossenem Magnetventil (208) das Magnetventil (207) geöffnet. Das mit Schmutzpartikeln angereicherte Reinigungsmittel (210) entweicht über die Entwässerungsleitung (206) in die Kanalisation. Eine weitere Reinigung kann mit Wasserspritzdüsen (211) vorgenommen werden, die Frischwasser über die Wärmeaustauscher (202) + (202) + (203) versprühen.

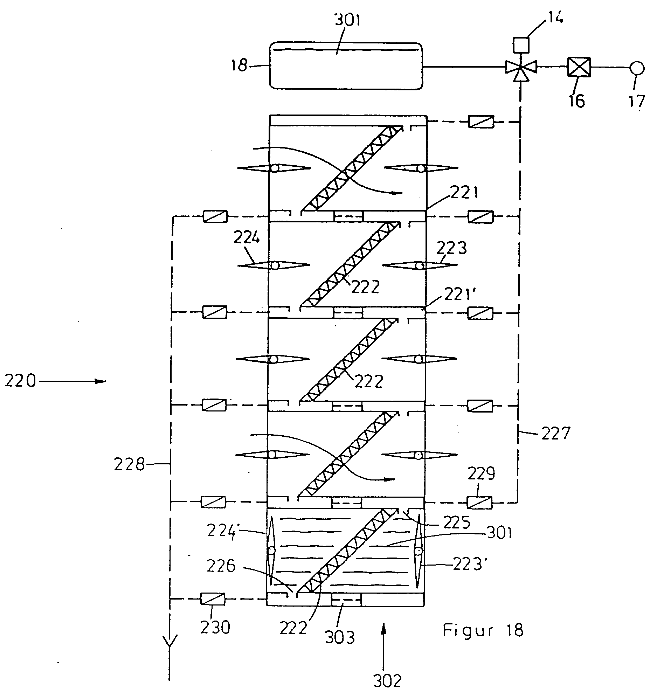

### Aufbau und Arbeitsweise Figur 18

Die Luftfilterreinigungseinrichtung (220) kann aber auch aus übereinander angeordneten wasserführenden Kanälen (221) bestehen, zwischen denen die Luftfilterkassetten (222) angeordnet sind. Zwischen den Kanalstücken (221) und (221') sind am Lufteintritt wasserdichte Luftabsperrklappen (223) und am Luftaustritt wasserdichte Luftabsperrklappen (224) installiert, die zusammen mit den Luftfilterkassetten (222) und den Kanälen (221) + (221') horizontal verlaufen. Die Kanäle (221) + (221') besitzen Wasseraustrittsöffnungen (225) und Wasserabströmöffnungen (226). Innerhalb der Kanäle (221) + (221') oder darüber sind Ultraschalleinrichtungen (226) installiert. Die rechte Seite der Kanäle (221) + (221') ist an eine Bewässerungsleitung (227) und die linke Seite an eine Entwässerungsleitung (228) angeschlossen.
An den Anschlüssen der Bewässerungsleitung (227) und Entwässerungsleitung (228) sind Magnetventile (229) und (300) installiert. Die Luftfilterreinigungseinrichtung (220) kann segmentweise beim Betrieb der Lüftungsanlage gereinigt werden. So z.B. sind in einem Segmentbereich die Luftabsperrklappen (223') + (224') geschlossen, während die Luftabsperrklappen (223) + (224) offen sind. Beim Reinigungsvorgang strömt Reinigungsmittel (301) z.B. in das untere Segment (302) über die Öffnungen (225) bei geschlossenen Absperrklappen (223') + (224') ein. Nach dem Reinigungsvorgang strömt das mit Schmutzpartikel behaftete Reinigungsmittel (301) über die Öffnung (226) ab. Anschließend öffnen sich die Luftabsperrklappen (223') + (224'). Der Reinigungsvorgang wiederholt sich kontinuierlich abschnittsweise von Segment zu Segment.

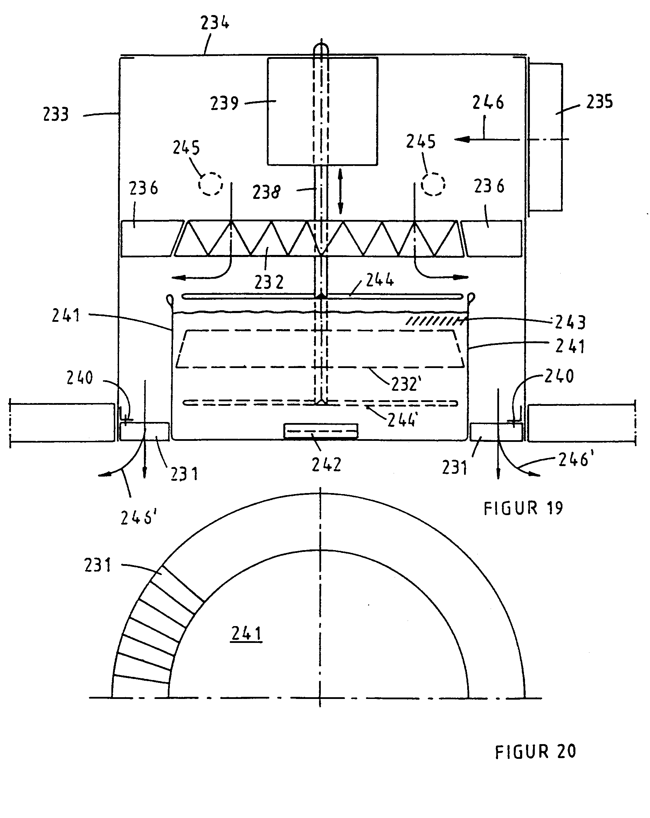

### Aufbau und Arbeitsweise Figur 19 + 20

Die Ausführung gem. Figur kann vereinfacht werden, wenn im Zuluftauslaß (230) anstelle der Kugeldüsen ( ) ein Drallring (231) deckenbündig installiert wird und wenn kein Filterring ( ), sondern eine Luftfilterscheibe (232) vorgesehen wird. Der Zuluftauslaß (230) besteht aus einem äußeren Verteilrohr (233), das einen oberen Abschlußdeckel (234) und einen Zuluftstutzen (235) besitzt. In der Mitte des Verteilrohres (233) ist eine Ringscheibe (236) angebracht, an die die Luftfilterscheibe (232) luftdicht angepreßt wird.
Die Filterscheibe (232) ist an einer Verstellstange (238) eines Stellmotors (239) befestigt, der an dem Deckel (234) montiert ist. Am unteren Ende des Verteilrohres (233) ist ein Drallring (231) an einer Haltevorrichtung (240) befestigt, von der der Drallring (231) sehr leicht gelöst werden kann.
Im Zentrum des Drallringes (231) befindet sich ein wasserdichter Reinigungstopf (241), der von dem Drallring (231) gehalten wird und oben offen ist. Der Topf (241) reicht bis etwa 20 - 40 mm unter die Ringscheibe (236). Am Boden des Reinigungstopfes (241) ist eine Ultraschalleinrichtung (242) befestigt. Im Reinigungstopf (241) befindet sich eine Reinigungsflüssigkeit (243), die bis kurz vor dem oberen Rand des Reinigungstopfes (241) aufgefüllt ist.
An dem unteren Ende der Verstellstange (238) ist eine Scheibe (244) montiert, die im Lüftungsbetrieb bündig zum oberen Rand des Reinigungstopfes (241) ist. Über der Luftfilterscheibe (232) sind UVC-Entkeimungslampen (245) angeordnet.

Beim Lüftungsbetrieb befindet sich die Luftfilterscheibe (232) in der Ringscheibe (236). Die Zuluft (246) strömt über die Luftfilterscheibe (232) und wird von der Scheibe (244) nach außen hin umgelenkt. Die gereinigte Zuluft (246') strömt anschließend über den Drallring (231) in den Raum.
Während der Nacht, z.B. bei Stillstand der Lüftung, drückt der Stellmotor (239) die Filterscheibe (232) zusammen mit der Scheibe (244) nach unten in das Reinigungsband (243). Nach längerem Einweichvorgang lösen sich die Schmutzpartikel von der Luftfilterscheibe (232).
Die Reinigungsflüssigkeit (243) wird außerdem von der Ultraschalleinrichtung (242) in Schwingungen versetzt, so daß sich auch im Inneren der Filterscheibe (232) die Restschmutzpartikel lösen.
Die Reinigungsflüssigkeit (243) kann auch über eine elektrische Heizeinrichtung je nach Filtermaterial bis auf 60 °C und bei Keramikfilter bis 100 °C erwärmt werden (Desinfektion).
Nach dem Einweich- und Reinigungsvorgang wird die Filterscheibe (232) bei eingeschalteten UVC-Entkeimungslampen (245) sehr langsam aus dem Reinigungsbad (243) gehoben und an die Ringscheibe (236) gepreßt.

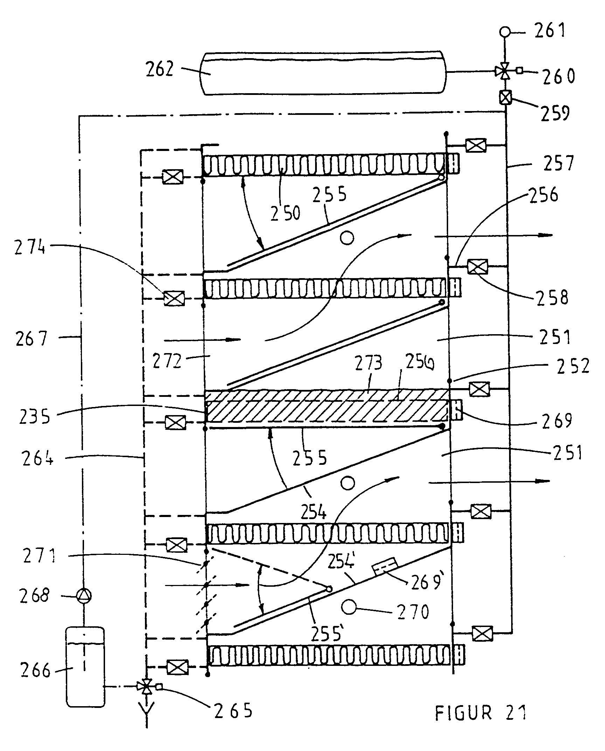

### Aufbau und Arbeitsweise Figur 21

Zur Minimierung der Füllmenge der Reinigungsflüssigkeit im Bereich der Filterkassette und der Erhöhung der Abscheideleistung über den Wert eines Elektrofilters können Microfilterpapiere verwendet werden, die nur eine Faltenhöhe von 40 mm besitzen und einen Durchlaßgrad von 2 % haben. Die Filterklasse kann damit auf R gesteigert werden. Die Filterkassette (250) aus gefaltetem Microfilterpapier ist dann in einem Kanalstück (251) vorzugsweise waagerecht untergebracht, das zur Waagerechten einen Winkel von etwa 20 Grad aufweist. Das Luftkanalstück (251) ist etwa 300 mm hoch und 600 mm breit. Das Kanalstück (251) ist mit einem rechten Stirnblech (252) und einem linken Stirnblech (253) bestückt, in dem die Microfilterkassette (250) luft- und wasserdicht befestigt ist. Die Microfilterkassette (250) ist auch an den beiden senkrechten Seitenwänden luft- und wasserdicht.
Zwischen der Microfilterkassette (250) und der unteren Wandung (254) des Luftkanals (251) ist eine einseitige drehbare,wasserdichte Absperrklappe (255) angeordnet, die sich über einen Stellmotor zwischen der Wandung (254) und der Unterkante der Microfilterkassette (250) verdrehen läßt. An das rechte Stirnblech (252) ist ein Rohrstutzen (256) angebracht, das mit einer Bewässerungsleitung (257) verbunden ist. In dem Rohrstutzen (256) ist ein Magnetventil (258) installiert. Die Bewässerungsleitung (257) ist in der schon eingangs beschriebenen Weise über einen Wassererwärmer (259) und ein Dreiwegeventil (260) an einen Frischwasseranschluß (261) und einen Reinigungsmittelbehälter (262) angeschlossen. An der linken Stirnseite (253) befindet sich ein Rohrstück (263), das an die Entwässerungsleitung (264) angeschlossen ist. Am Ende der Entwässerungsleitung (264) kann auch ein Dreiwegeventil (265) installiert sein, das mit einem Sammelbehälter (266) verbunden ist.
Der Sammelbehälter (266) ist über die Rohrleitung (267) an die Bewässerungsleitung (257) angeschlossen. In der Rohrleitung (267) befindet sich eine Pumpe (268). An dem Stirnblech (252) sind Ultraschalleinrichtungen (269) installiert. oberhalb der Microfilterkassetten (250) können UVC-Entkeimungslampen (270) installiert sein, Die wasserdichte Absperrklappe (255') kann auch auf der Mitte der Kanalwandung (254') drehbar gelagert sein. Eine weitere Möglichkeit besteht darin, eine senkrechte wasserdichte Absperrklappe (271) am Lufteintritt (272) des Kanalstücks (251) zu installieren. In beiden Fällen ist es von Vorteil, wenn die Ultraschalleinrichtung (269') auf der Wandung (254') plaziert wird.
Die Reinigugnsvorgänge sind ähnlich wie eingangs beschrieben. Die wasserdichte Absperrklappe (255') wird mit Hilfe eines Stellmotors gegen die Microfilterkassette (250) gepreßt. Damit wird der Luftdurchgang durch die Öffnung (272) bzw. das Luftkanalstück (251) unterbunden.
Das Magnetventil (258) öffnet den Wasserzufluß vor dem Frischwasseranschluß (261) oder dem Reinigungsmitteltank (262). Die Microfilterkassette (250) wird von Frischwasser oder Reinigungsmittel (273) überflutet. Der Einweichvorgang findet statt, die Schmutzpartikel lösen sich von der Microfilterkassette (250). Der Reinigungsvorgang wird noch zusätzlich gesteigert, wenn die Ultraschalleinrichtung (269) bzw. (269') in Betrieb genommen wird, die das Reinigungsmittel (273) in Schwingungen versetzt. Nach dem Einweichvorgang der eine oder mehrere Stunden dauern kann, wird das Magnetventil (274) geöffnet. Das Reinigungsmittel (273) fließt über die Entwässerungsleitung (264) ab oder kann über den Sammelbehälter (266),die Pumpe (268) und die Rohrleitung (267) im Kreislauf gehalten werden.
Ist das Reinigungsmittel (273) abgeflossen, dann kann mittels der UVC-Entkeimungslampen (270) die Microfilterkassette (250) getrocknet und noch zusätzlich entkeimt werden. Die Trocknung kann aber auch über einen Heizstab oder über Heißluft vorgenommen werden.
Bei der Ausführung mit der Absperrklappe (255') ist das Reinigungsbad größer. Das Reinigungsbad wird noch größer, wenn eine senkrechte Absperrklappe (271) vorhanden ist. In beiden Fällen wird das Reinigungsbad über die Ultraschalleinrichtung (269') auf direktem Wege in Schwingungen versetzt. Die beschriebenen Reinigungsvorgänge können abschnittsweise pro Kanalstück (251) während des Betriebes der Lüftungsanlage vorgenommen werden.

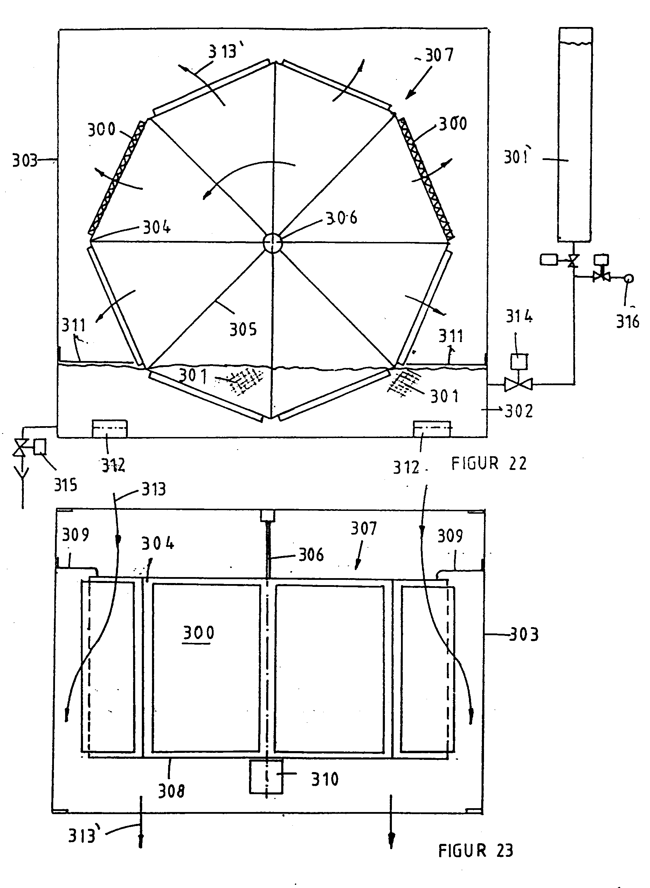

### Aufbau Figur 22 + 23

Das Regenerationsluftfilter kann aber auch ohne wasserdichte Luftabsperrklappen ausgebildet sein. In diesem Falle muß das Luftfiltermaterial in Rotation versetzt werden, damit die Filterkassetten (300) in ein Reinigungsmittel (301) tauchen, das sich in einer Wanne (302) am Boden eines Luftkanals (303) befindet. Die Luftfilterkassetten (300) sind auf der umlaufenden Seite an der Außenwandung (304), die mehreckig, z.B. achteckig, sein kann, aufgeschraubt. Von den Außenwandungen (304) verlaufen Trennbleche (305), die mit einer drehbaren Welle (306) verbunden sind. Das drehbare Luftfilterrad (307) ist mit einer Abschlußplatte (308) versehen, die luftdicht ist. Die Vorderseite des Luftfilterrades (307) ist mit einer Lochscheibe (309) abgedichtet und mit dem Luftkanal (303) verbunden. Die Welle (306) ist mit einem Elektromotor (310) verbunden, der das Luftfilterrad (307) antreibt bzw. verdreht. Über dem in der Wanne (302) befindlichen Reinigungsmittel (301) sind Abdeckbleche (311) + (311') angeordnet.

### Betriebsweise Figur 22 + 23

In Abhängigkeit von der Verschmutzung der Luftfilterkassetten (300) oder in einem gleichmäßigen Turnus wird das Luftfilterrad (307) über dem Elektromotor (310) verdreht, so daß sich immer zwei Filterkassetten (300) im Reinigungsbad (301) befinden.
Das Reinigungsbad (301) kann dann noch von den Ultraschalleinrichtungen (312) zur besseren Schmutzlösung in Schwingungen versetzt werden. Die verschmutzte Außenluft (313) strömt vorne ein, durchströmt die Luftfilterkassetten (300) und gelangt als gereinigte Außenluft (313') zum Klimazentralgerät. Das Reinigungsmittel (301) kann über die Magnetventile (314) + (315) gefüllt und entleert werden. Die Wanne (302) kann abwechselnd mit Reinigungsmittel (301') oder auch Stadtwasser (316) beschickt werden. Diese Ausführung ist auch für die Luftbehandlung in Fabrikationseinrichtungen geeignet.

## Patentansprüche

1. Verfahren zum Reinigen eines Filters, insbesondere eines in einen Strömungskanal eingebauten Filters, wie des Luftfilters einer Klimaanlage, **dadurch gekennzeichnet, daß** das Filter in sporadischen Abständen einem Reinigungsvorgang unterzogen wird, wobei es mit einem flüssigen Reinigungsmittel beaufschlagt wird, mit welchem danach die Verunreinigungen abgeführt werden.

2. Verfahren nach Anspruch 1, **dadurch gekennzeichnet, daß** das Filter zumindest zu Beginn des Reinigungsvorgangs mit dem Reinigungsmittel besprüht wird.

3. Verfahren nach einem der vorhergehenden Ansprüche, **dadurch gekennzeichnet, daß** das Filter mit dem Reinigungsmittel überflutet wird und daß beim späteren Entfluten die Verunreinigungen mit dem Reinigungsmittel abgeführt werden.

4. Verfahren nach einem der vorhergehenden Ansprüche, **dadurch gekennzeichnet, daß** das Filter über eine vorgegebene Einweichzeit im Reinigungsmittel eingeweicht bleibt.

5. Verfahren nach einem der vorhergehenden Ansprüche, **dadurch gekennzeichnet, daß** das Filter im Anschluß an einen Reinigungsvorgang wenigstens einmal mit einem Spülmittel gespült wird.

6. Verfahren nach einem der vorhergehenden Ansprüche, **dadurch gekennzeichnet, daß** das Filter im Anschluß an einen Reinigungs- und/oder Spülvorgang getrocknet und/oder desinfiziert wird.

7. Verfahren nach einem der vorhergehenden Ansprüche, **dadurch gekennzeichnet, daß** das Reinigungsmittel während des Reinigungsvorgangs, insbesondere während der Einweichzeit, zu Bewegungen, insbesondere Schwingungen angeregt ist.

8. Verfahren nach einem der vorhergehenden Ansprüche, **dadurch gekennzeichnet, daß** das Reinigungsmittel erwärmt wird.

9. Vorrichtung zur Durchführung des Verfahrens nach wenigstens einem der vorhergehenden Ansprüche, **dadurch gekennzeichnet, daß** dem zu reinigenden Filter ein wannenförmiger Behälter zugeordnet ist, in den eine auf- und absteuerbare Zulaufleitung mündet und von dem eine auf- und absteuerbare Abflußleitung und vorzugsweise eine Überlaufleitung abgeht.

10. Vorrichtung nach Anspruch 9, **dadurch gekennzeichnet, daß** an die Zulaufleitung wenigstens ein mit Sprühdüsen versehener, im Behälter angeordneter Düsenbalken angeschlossen ist.

11. Vorrichtung nach einem der vorhergehenden Ansprüche 9 oder 10, **dadurch gekennzeichnet, daß** ein Strömungskanal über dem Kanalquerschnitt mehrere übereinander angeordnete Kanalabschnitte aufweist, die jeweils einen Filterabschnitt mit zugeordnetem Behälter enthalten.

12. Vorrichtung nach einem der vorhergehenden Ansprüche 9 bis 11, **dadurch gekennzeichnet, daß** der Filterabschnitt mit etwa waagrechter Ausrichtung diagonal im zugeordneten Kanalabschnitt angeordnet ist, dessen Achse gegenüber der Horizontalen geneigt ist, und daß wenigstens eine schwenkibare Klappe vorgesehen ist, mittels welcher der Strömungsweg zwischen dem Filterabschnitt und dem tiefer angeordneten öffnungsquerschnitt des zumindest im Bereich des zugeordneten Filterabschnitts flüssigkeitsdichten, an die Zulaufleitung, die Abflußleitung und vorzugsweise Überlaufleitung angeschlossenen Kanalabschnitts flüssigkeitsdicht abdichtbar ist.

13. Vorrichtung nach einem der vorhergehenden Ansprüche 9 bis 12, **dadurch gekennzeichnet, daß** der Behälter mit einem vorzugsweise auf der Seite der Einmündung der Zulaufleitung angeordneten Ultraschgallwellengenerator versehen ist.

14. Vorrichtung nach einem der vorhergehenden Ansprüche 9 bis 13, **dadurch gekennzeichnet, daß** die Zulaufleitung wahlweise an eine Reinigungsmittelquelle oder eine Spülmittelquelle anschließbar ist.
